(19) Europäisches Patentamt
European Patent Office
Office européen des brevets

(11) Publication number: **0 156 644**
**A1**

(12) # EUROPEAN PATENT APPLICATION

(21) Application number: 85302066.7

(22) Date of filing: 26.03.85

(51) Int. Cl.[4]: C 07 D 233/64
C 07 C 103/30

(30) Priority: 02.04.84 US 596216

(43) Date of publication of application:
02.10.85 Bulletin 85/40

(84) Designated Contracting States:
AT BE CH DE FR GB IT LI LU NL SE

(71) Applicant: PFIZER INC.
235 East 42nd Street
New York, N.Y. 10017(US)

(72) Inventor: Reiter, Lawrence Alan
13 Washington Drive
Gales Ferry Connecticut(US)

(74) Representative: Graham, Philip Colin Christison et al,
Pfizer Limited Ramsgate Road
Sandwich, Kent CT13 9NJ(GB)

(54) **Process and intermediates for 4-acetylimidazoles.**

(57) This invention provides a two-step process for the preparation of 4-acetylimidazoles. In the first step, a 4-acylamino-5-methylisoxazole is subjected to catalytic hydrogenolysis to give a 4-amino-3-acyl-amino-3-buten-2-one. In the second step, the intermediate 3-buten-2-one compound is cyclized to a 4-acetylimidazole by treatment with a basic agent. The 4-acetylimidazoles are chemical intermediates for preparing known antisecretory agents and histamine $H_2$ antagonists, useful for the treatment of gastric hyperacidity and peptic ulcers.

EP 0 156 644 A1

# PROCESS AND INTERMEDIATES FOR 4-ACETYLIMIDAZOLES

This invention provides a new chemical process, and novel chemical compounds used therein. More particularly, the new chemical process of this invention involves the rearrangement of a 4-acylamino-5-methylisoxazole compound to a 2-(optionally-substituted)-4-acetylimidazole compound. The novel chemical compounds used in the process of this invention are derivatives of 4-amino-3-buten-2-one ($NH_2$-CH=CH-CO-$CH_3$), having an acylamino substituent ($R^1$-CO-NH-) at the 3-position.

The 4-acetylimidazoles produced by the process of this invention are known chemical intermediates useful in the preparation of certain 4-(2-guanidino-4-thiazolyl)imidazole compounds. Said 4-(2-guanidino-4-thiazolyl)imidazoles are known from United States Patent No. 4,374,843 to have value as medicinal agents in the treatment of disorders caused by, or aggravated by, the secretion of gastric acid, e.g. peptic ulcers.

Various 4-acetylimidazoles have previously been prepared by irradiation of an *N*-acetylimidazole, and by condensation of a 3-halo-4-*n*-alkoxy-3-buten-2-one with an amidine in the presence of a base. See further, United States Patent No. 4,374,843; LaMattina *et al*., *Journal of Organic Chemistry*, *48*, 897 (1983); and Lipinski *et al*., *Journal of Organic Chemistry*, *49*, 566 (1984). Ajello (*Annali di Chimica* [Rome], *60*, 343 [1970]) has described the preparation of certain 2,5-disubstituted-4-acetylimidazoles from isoxazoles by hydrogenolysis followed by a base-catalyzed cyclization.

This invention provides a new process for the preparation of an imidazole compound of the formula

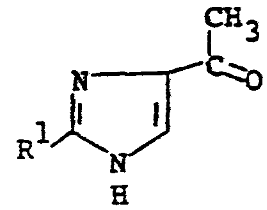

---(I)

which comprises the steps of

(a) contacting an isoxazole compound of the formula

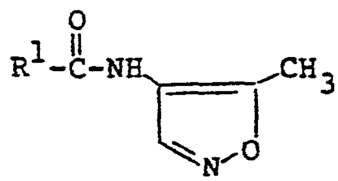

---(II)

with hydrogen, in a reaction-inert solvent, in the presence of a catalytic amount of a hydrogenolysis catalyst, to give an intermediate compound of the formula

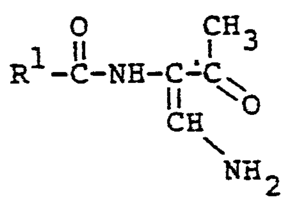

---(III)

and (b) cyclizing the intermediate compound from step (a) by treating said intermediate compound with a basic agent having a $pK_b$ greater than 9, in a reaction-inert solvent;

wherein $R^1$ is selected from the group consisting of hydrogen, alkyl having 1 to 6 carbons, hydroxymethyl and $-(CH_2)_n-Ph$; wherein n is an integer from 2 to 4, and "Ph" represents the phenyl group.

Also embraced within this invention are the novel chemical compounds of the formula III, wherein $R^1$ is as defined previously.

Preferred embodiments of this invention are the aforesaid two-step process, in which $R^1$ is hydrogen, methyl or hydroxymethyl, especially methyl; and also the compounds of formula III, in which $R^1$ is hydrogen, methyl or hydroxymethyl, especially methyl.

Step (a) of the process of this invention involves contacting a compound of formula II, wherein $R^1$ is as defined previously, with hydrogen, in a reaction-inert solvent, in the presence of a catalytic amount of a hydrogenolysis catalyst. This is usually accomplished by stirring or shaking a solution of a compound of formula II and the catalyst under an atmosphere of hydrogen, or hydrogen mixed with up to 50% by volume of an inert diluent such as nitrogen or argon, in a closed vessel, until conversion into a compound of formula III is substantially complete. Preferably, step (a) is carried out under an atmosphere consisting substantially of hydrogen, i.e. at least 90% hydrogen by volume, preferably at a pressure in the range from 0.5 to 10 $kgcm^{-2}$.

Reaction-inert solvents which can be used for this reaction are those which do not adversely interact with either the compound of formula II or the compound of formula III, do not react to any significant degree with hydrogen, and will dissolve the compound of formula II to a substantial degree. Additionally, it is desirable that the solvent be volatile enough to be removed by an evaporative process or be appreciably soluble in water. Typical solvents which can be used

include: lower-alkanols, such as methanol, ethanol and n-butanol; low-molecular weight ketones, such as acetone and methyl isobutyl ketone; low-molecular weight esters, such as ethyl acetate; low-molecular weight open-chain ethers, such as 1,2-dialkoxyethanes (e.g., 1,2-dimethoxyethane) and di(2-alkoxyethyl) ethers (e.g., di[2-methoxyethyl] ether); low-molecular weight cyclic ethers, such as tetrahydrofuran and dioxane; and mixtures of these solvents. The solvent system for step (a) can be partially aqueous, but it is usual to choose a homogeneous solvent system, and also one which dissolves substantially all of the compound of formula II.

Step (a) is preferably carried out at a temperature in the range from 0 to 80°C., but more preferably at a temperature from 15 to 30°C.

Step (a) of the process of this invention entails cleavage of a nitrogen-oxygen bond by hydrogenolysis, and the catalysts which can be used are those which are commonly used in the art for this type of transformation. In particular catalysts which can be used are transition metals, such as palladium, platinum and nickel and mixtures thereof. The hydrogenolysis catalyst is used in an amount which is commonly used in the art in this type of transformation, and in this specification this is referred to as a "catalytic amount." Such an amount is normally in the range from 1 to 10 percent by weight, especially 3 to 5 percent, based on the weight of the starting isoxazole of formula II.

For ease of operation, it is often convenient to use the hydrogenolysis catalyst suspended on an inert carrier. In such a situation, the ratio of hydrogenolysis catalyst to carrier will normally be in the range

from 1:40 to 1:5, and preferably about 1:10, by weight. Typical carriers which can be used are powdered carbon and calcium carbonate. A 10% suspension of palladium-on-carbon is a particularly convenient catalyst for step (a) of this invention.

Step (a) of the process of this invention is usually carried out under neutral conditions. However, reaction conditions having a pH in the range from 4 to 8 can be used satisfactorily.

Step (a) normally proceeds quite rapidly, and, when carried out under the preferred conditions, conversion of the isoxazole of formula II into the compound of formula III is normally complete within about 0.5 to about 4 hours.

Step (b) of the process of this invention involves cyclizing the product of step (a), i.e., the compound of formula III, by treatment with a basic agent in a reaction-inert solvent.

Reaction-inert solvents which can be used for step (b) are those solvents which do not adversely interact with the starting compound of formula III or the product of formula I, are not substantially affected by the basic agent used for step (b), and will dissolve the starting compound of formula III to a significant degree. Additionally, the solvent should be such that the product imidazole of formula I can be recovered readily. In general, the reaction-inert solvent should be sufficiently volatile that it can be removed by evaporation, or it should be water-miscible. Typical solvents which can be used include lower-alkanols, such as methanol, ethanol and n-butanol; the low-molecular weight open-chain and cyclic ethers mentioned previously for step (a); water; and mixtures of these solvents.

A wide variety of basic agents can be used for step (b) of this invention, the major requirements being that the basic agent does not adversely affect either the starting material of formula III, or the product of formula I, and it has sufficient basic strength to bring about the desired cyclization. In this regard, basic agents having a $pK_b$ value of greater than 9 are required. Moreover, as will be appreciated by one skilled in the art, the basic agent chosen must be compatible with the solvent system chosen for step (b).

In practice, convenient basic agents for step (b) of this invention are alkaline metal hydroxides, such as sodium hydroxide and potassium hydroxide; alkaline earth metal hydroxides, such as barium hydroxide; alkali metal alkoxides having 1 to 5 carbons, such as sodium ethoxide and potassium t-butoxide; alkali metal hydrides, such as sodium hydride and potassium hydride; and alkaline earth metal hydrides, such as calcium hydride.

As regards the amount of basic agent to be used in step (b), at least one molar equivalent is required, and amounts in excess of one molar equivalent can be used. In practice, an amount in the range from one molar equivalent to six molar equivalents, and preferably one to two molar equivalents, of basic agent are used.

Step (b) of the process of this invention is normally carried out at a temperature in the range from 60 to 120°C., and preferably from 70 to 100°C. Within this preferred temperature range, the cyclization step is normally essentially complete within a few hours, e.g., 1 to 4 hours.

If desired, the intermediate 3-buten-2-one compound of the formula III can be isolated at the end of step (a). This is conveniently accomplished by firstly removing the catalyst by filtration, to give a solution of the compound of formula III. If the solvent in which step (a) has been carried is sufficiently volatile, removal of the solvent by evaporation, preferably in vacuo, then affords the compound of formula III. Alternatively, when the solvent in which step (a) has been carried out is miscible with water, a convenient method of isolating the compound of formula III involves dilution of the solution of formula III with water, extraction with a volatile, water-immiscible, organic solvent, followed by solvent evaporation.

Alternatively, however, if desired, the intermediate 3-buten-2-one of formula III need not be isolated. At the end of step (a), after removal of the catalyst by filtration, the resulting solution of the compound of formula III can be used directly in step (b). This is achieved simply by adding the appropriate amount of a basic agent to the solution of the compound of formula III, and carrying out the cyclization directly.

As will be appreciated by those skilled in the art, when it is desired to carry out steps (a) and (b) of the process of this invention without isolation of the compound of formula III, it is necessary to conduct step (a) in a solvent which is compatible with the cyclization conditions of step (b).

At the conclusion of step (b), the final 4-acetylimidazole compound of formula I can be isolated by standard techniques. For example, the basic agent can be neutralized, and then if a volatile solvent

has been used, it can be removed by evaporation *in vacuo* to give the crude product. If inorganic salts are still present, they can be removed by extracting the product into an organic solvent such as acetone, filtering off the inorganic salts, and recovering the compound of formula I by evaporation. Alternatively, after neutralization of the basic agent, the reaction mixture can be diluted with water. If the reaction solvent forms a separate layer, it can be removed, dried and evaporated to give the desired compound of formula I. If two phases are not present, the product can be extracted into a water-immiscible, volatile organic solvent. Evaporation of the organic solvent then affords the desired compound of formula I.

The compound of formula I can be purified by standard techniques, such as recrystallization and chromatography, if desired.

The starting isoxazoles of formula II can be prepared simply by acylation of 4-amino-5-methylisoxazole (IV), using an activated derivative of a carboxylic acid of the formula $R^1$-CO-OH, wherein $R^1$ is as defined previously, viz:

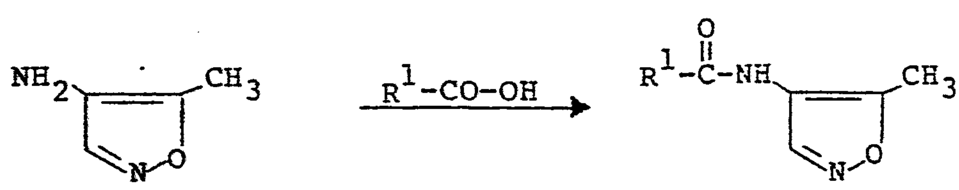


IV II

Conversion of compound IV into compound II is a classic acylation reaction, and it can be carried out under standard conditions, well known in the art for

this type of process. The acid of formula $R^1$-CO-OH can be activated by standard techniques such as conversion to an acid chloride of the formula $R^1$-CO-Cl, a symmetrical anhydride of the formula $(R^1\text{-CO})_2O$, or a mixed carboxylic-carbonic anhydride of the formula $R^1$-CO-O-CO-O$R^2$, wherein $R^2$ is a lower-alkyl group, e.g., isobutyl.

The 4-acetylimidazole compounds of formula I are useful as intermediates to known antisecretory agents and histamine $H_2$ antagonists of the formula

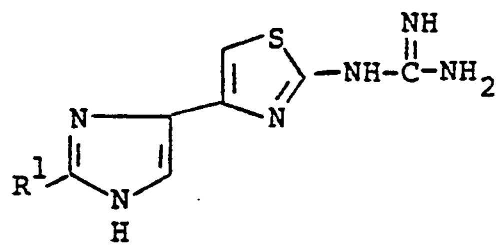

---(V)

wherein $R^1$ is as defined previously. Conversion of the compounds of formula I into the compounds of formula V, and methods for the use of the compounds of formula V in the treatment of conditions caused by gastric hyperacidity (e.g., peptic ulcers), are taught in United States Patent No. 4,374,843.

For example, the compounds of formula I are first converted into the corresponding 4-haloacetylimidazole of the formula VI by reaction with a halogen in an aqueous hydrogen halide solution, e.g., bromine in aqueous hydrobromic acid, viz:

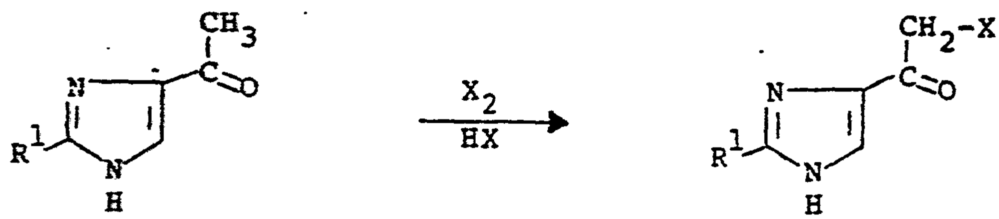


(I) (VI)

wherein $R^1$ is as defined previously and X is halo, e.g., bromo. The compound of formula VI is then reacted with N-guanylthiourea (VII) to give a compound of the formula V:

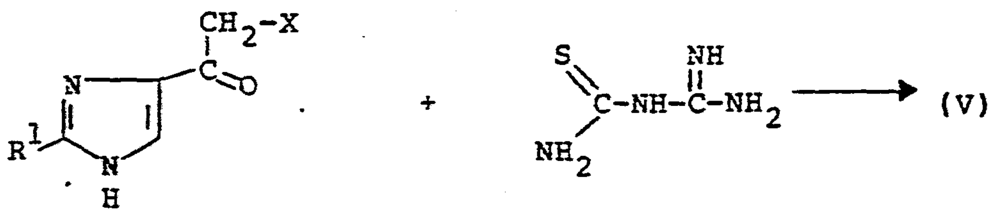


(VI) (VII)

The following examples and preparations are being provided solely for the purpose of further illustration. Proton nuclear magnetic reasonance spectra (NMR spectra) were measureed at 60 MHz in deuterochloroform ($CDCl_3$), perdeuteroacetone ($CD_3COCD_3$), perdeuteromethanol ($CD_3OD$) or perdeuterodimethyl sulfoxide (DMSO-$d_6$), and the following abbreviations for peak shapes are used: s, singlet; d, doublet; t, triplet; and q, quartet. Peak positions are reported in parts per million (ppm) downfield from internal tetramethylsilane. For mass spectra, the peak positions are reported as mass-to-charge ratios (m/e) with the relative intensities being shown in parenthesis after

the peak positions, expressed as a percentage of the most intense peak. The term "flash column chromatography" refers to the procedure described by Still et al., Journal of Organic Chemistry, 43, 2923 (1978).

## EXAMPLE 1

### 4-Acetylimidazole

A solution of 715 mg (5.67 mmole) of 4-formamido-5-methylisoxazole in ethanol was shaken under an atmosphere of hydrogen, at ca. 4 kg/cm$^2$, in the presence of 200 mg of 10% palladium-on-carbon, for 30 minutes. The reaction mixture was filtered and the residue was washed with ethanol. The filtrate and the washings were combined, and to the resulting solution was added 284 mg (7.1 mmole) of solid sodium hydroxide. The resulting mixture was heated under reflux for 1 hour, and then 418 mg (7.8 mmole) of solid ammonium chloride was added. The mixture was cooled to room temperature and the solvent was removed by evaporation *in vacuo*. The residue was extracted with acetone, and the resulting acetone solution was evaporated *in vacuo* to give the crude product. The crude product was purified by flash column chromatography to give 610 mg (98% yield) of the title compound as a white solid, mp 169-170°C.

The NMR spectrum of the product ($CD_3COCD_3$/DMSO-$d_6$) showed absorptions at 2.45 (s, 3H) and 7.67 (s, 2H) ppm.

The mass spectrum of the product showed peaks at m/e values of 110 (100), 95 (88.4), 81 (3.3), 68 (32.4) and 67 (36.6).

## EXAMPLE 2

### 4-Acetyl-2-methylimidazole

A solution of 468 mg (3.34 mmole) of 4-acetamido-5-methylisoxazole in 20 ml of ethanol was shaken under an atmosphere of hydrogen, at a pressure of 2 $kg/cm^2$, in the presence of 100 mg of 10% palladium-on-carbon, for 1.5 hours. A slurry of 100 mg of 10% palladium-on-carbon and 145 mg of non-pyrophoric Raney nickel in a few milliliters of ethanol was then added, and the resulting mixture was shaken under hydrogen, at 2.0 $kg/cm^2$ pressure, for a further 1 hour. The reaction mixture was filtered and the residue was washed with ethanol. The filtrate and the washings were combined and heated to reflux temperature. To the refluxing solution was added 153 mg of solid sodium hydroxide. The mixture was heated at reflux temperature for 1 hour, and then an additional 95 mg of solid sodium hydroxide was added. The mixture was heated at reflux temperature for an additional 30 minutes, and then 365 mg of solid ammonium chloride was added. The resulting mixture was allowed to stand overnight and then the solvent was removed by evaporation *in vacuo*. The residue was extracted with acetone, and the resulting acetone solution was evaporated *in vacuo*, to give 420 mg of a yellow solid. The yellow solid was recrystallized from a mixture of diisopropyl ether and ethyl acetate (1:1) to give 153 mg (37% yield) of the title compound, mp 128-130°C.

EXAMPLE 2 (Cont.)

The mother liquor from the recrystallization was evaporated in vacuo, and the residue was purified by flash column chromatography. This gave an additional 171 mg (41% yield) of the title compound, mp 123-127°C.

Total yield: 324 mg (78% yield).

The NMR spectrum of the product (DMSO-$d_6$) showed absorptions at 2.33 (s, 3H), 2.38 (s, 3H) and 7.68 (s, 1H) ppm.

The mass spectrum of the product showed peaks at m/e value of 124 (47.5), 109 (100), 81 (98.1) and 54 (68.3).

EXAMPLE 3

4-Acetyl-2-ethylimidazole

A solution of 1.13 g (7.3 mmole) of 4-propionamido-5-methylisoxazole in 20 ml of ethanol was shaken under an atmosphere of hydrogen, at a pressure of ca 4 $kg/cm^2$, in the presence of 200 mg of 10% palladium-on-carbon, for 30 minutes. At this point, an additional 200 mg of 10% palladium-on-carbon was added and the hydrogenation was continued for an additional 30 minutes at ca 4 $kg/cm^2$. The reaction mixture was filtered, and the residue was washed with ethanol. To the combined filtrate and ethanol washings was added 356 mg (8.8 mmole) of solid sodium hydroxide. The resulting mixture was stirred at room temperature for 1 hour and then under reflux for 1 hour. To the hot solution was added 518 mg (9.7 mmole) of solid ammonium chloride and the mixture was allowed to cool to room temperature. The solvent was removed by evaporation *in vacuo*, and the residue was extracted with acetone. The resulting acetone solution was evaporated *in vacuo* to give a yellow solid. The yellow solid was purified by flash column chromatography to give the title compound as a white solid, 878 mg, mp 117-119°C. (87% yield).

The NMR spectrum of the product ($CDCl_3$) showed absorptions at 1.37 (t, 3H, J = 7Hz), 2.52 (s, 3H), 2.88 (q, 2H, J = 7Hz) and 7.70 (s, 1H) ppm.

The mass spectrum of the product showed peaks at m/e value of 138 (100), 123 (93.8), 95 (48.8), 81 (12.7) and 68 (28.3).

## EXAMPLE 4

### 3-Acetamido-4-amino-3-buten-2-one

A solution of 281 mg (2.0 mmole) of 4-acetamido-5-methylisoxazole in 25 ml of methanol was shaken under an atmosphere of hydrogen, at a pressure of ca 4 kg/$cm^2$, in the presence of 100 mg of 10% palladium-on-carbon for 1 hour. The catalyst was removed by filtration, and then the methanol solvent was removed by evaporation in vacuo. This afforded 280 mg of the title compound mp 157-160°C.

The NMR spectrum of the product (DMSO-$d_6$) showed absorptions at 1.95 (s, 3H), 2.00 (s, 3H), 6.40 (broad d, 2H), 7.35 (broad t, 1H) and 8.25 (broad s, 1H) ppm.

The mass spectrum of the product showed peaks at m/e values of 142 (72.2), 100 (100), 85 (24.1), 72 (35.7), 57 (66.0) and 43 (86.8).

## PREPARATION 1

### 4-Formamido-5-methylisoxazole

A solution of 1.08 g (8.0 mmole) of 4-amino-5-methylisoxazole hydrochloride in 3.0 ml of formic acid was heated under reflux for 7 hours, and then the reaction mixture was allowed to stand overnight. The formic acid was then removed by evaporation *in vacuo*, and the residue was purified by flash column chromatography and dried under high vacuum to give 717 mg (71% yield) of the title compound as an off-white solid, mp 73-75°C.

The NMR spectrum of the product ($CD_3OD$) showed absorptions at 2.42 (s, 3H), 8.18 (s, 1H) and 8.57 (s, 1H) ppm.

The mass spectrum of the product showed peaks at m/e value of 127 (1.3), 126 (1.6), 111 (0.3), 98 (1.1), 84 (0.4), 71 (6.6), 56 (10.1) and 43 (100).

Analysis: - Calcd. for $C_5H_6N_2O_2$:

C, 47.62; H, 4.80; N, 22.21%.

Found: C, 47.50; H, 4.80; N, 22.30%.

## PREPARATION 2

### 4-Acetamido-5-methylisoxazole

A mixture prepared from 1.35 g (10.0 mmole) of 4-amino-5-methylisoxazole hydrochloride, 820 mg (10.0 mmole) of sodium acetate, 5 ml of acetic anhydride and 45 ml of acetic acid was stirred at room temperature for 2 hours. The volatile components were then removed by evaporation *in vacuo*, and the residue was dissolved in diethyl ether. The ethereal solution was dried ($MgSO_4$) and then the solvent was removed *in vacuo*. The residue was dried under high vacuum, giving 1.38 g (98% yield) of the title compound as a white solid, mp 94-97°C.

The NMR spectrum of the product ($CDCl_3$) showed absorptions at 2.17 (s, 3H), 2.40 (s, 3H), 8.57 (s, 1H) and 8.73 (broad s, 1H) ppm.

The mass spectrum of the product showed peaks at m/e value of 140 (11.1), 98 (49.4), 71 (27.4) and 43 (100).

## PREPARATION 3

### 4-Propionamido-5-methylisoxazole

A mixture prepared from 1.35 g (10.0 mmole) of 4-amino-5-methylisoxazole hydrochloride, 961 mg (10.0 mmole) of sodium propionate, 6.51 g (50 mmole) of propionic anhydride and 10 ml of propionic acid was stirred at room temperature for 4 hours. The reaction mixture was then diluted with 100 ml of water, and extracted liberally with chloroform. The combined organic extracts were washed with sufficient aqueous potassium carbonate to keep the aqueous phase alkaline. The layers were separated and the aqueous phase was extracted with chloroform. The combined chloroform solutions were dried ($MgSO_4$) and evaporated *in vacuo*, with final traces of propionic anhydride being removed under high vacuum. The residue was purified by flash column chromatography to give 1.36 g (88% yield) of the title compound as a pale yellow oil, which crystallized on standing, mp 56-58°C.

The NMR spectrum of the product ($CDCl_3$) showed absorptions at 1.22 (t, 3H, J = 7Hz), 2.35 (s, 3H), 2.36 (q, 2H, J = 7Hz), 8.33 (broad s, 1H) and 8.47 (s, 1H) ppm.

The mass spectrum of the product showed peaks at m/e values of 155 (100), 154 (46.4), 138 (7.5), 112 (19.9), 98 (9.1), 71 (19.1) and 57 (89.5).

Analysis: - Calcd. for $C_7H_{10}N_2O_2$:

C, 54.53; H, 6.54; N, 18.17%.

Found: C, 54.28; H, 6.31; N, 17.99%.

## PREPARATION 4

### 4-Amino-5-methylisoxazole

The title compound can be prepared by nitration of 5-methylisoxazole using fuming nitric acid in fuming sulfuric acid, according to the procedure of Quilico and Musante, Gazz Chim. Ital., 71, 327 (1941), followed by reduction with aluminum amalgam, according to the procedure of Morgan and Burgess, J. Chem. Soc. 697 (1921).

CLAIMS

FOR ALL DESIGNATED COUNTRIES EXCEPT AUSTRIA

1. A process for the preparation of an imidazole compound of the formula

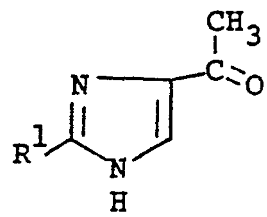

--- (I)

which comprises the steps of

(a) contacting an isoxazole compound of the formula

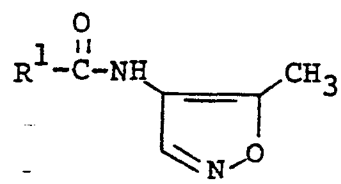

--- (II)

with hydrogen, in a reaction-inert solvent, in the presence of a catalytic amount of a hydrogenolysis catalyst, to give an intermediate compound of the formula

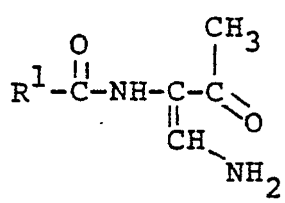

--- (III)

and (b) cyclizing the intermediate compound from step (a) by treating said intermediate compound with a basic agent having a $pK_b$ greater than 9, in a reaction-inert solvent;

wherein $R^1$ is hydrogen, alkyl having 1 to 6 carbons, hydroxymethyl or $-(CH_2)_n-Ph$, wherein n is an integer from 2 to 4 and Ph represents the phenyl group.

2. The process according to claim 1, wherein the basic agent used in step (b) is selected from the group consisting of alkali metal hydroxides, alkaline earth metal hydroxides, alkali metal alkoxides having 1 to 5 carbons, alkali metal hydrides and alkaline earth metal hydrides.

3. The process according to claim 2, wherein $R^1$ is hydrogen, methyl or hydroxymethyl.

4. The process according to claim 3, wherein step (a) is carried out under an atmosphere consisting substantially of hydrogen, at a temperature in the range from 0 to 80°C., at a pressure in the range from 0.5 to 10 $kg/cm^2$ and at a pH in the range from 4 to 8, and said hydrogenolysis catalyst is palladium, platinum or nickel.

5. The process according to claim 4, wherein the hydrogenolysis catalyst is present in an amount in the range from 1 to 10 weight-percent, based on the weight of said isoxazole.

6. The process according to claim 5, wherein the hydrogenolysis catalyst is palladium and it is suspended on powdered carbon, such that the ratio of catalyst to powdered carbon is in the range from 1:40 to 1:5 by weight.

7. The process according to claim 4, wherein step (b) is carried out at a temperature in the range from 60 to 120°C., and said basic agent used in step (b) is sodium hydroxide, potassium hydroxide or calcium hydroxide.

8. A compound of the formula

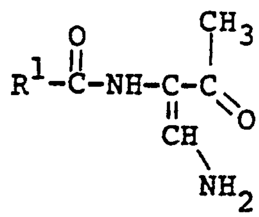

--- (III)

wherein $R^1$ is hydrogen, alkyl having 1 to 6 carbons, hydroxymethyl or $-(CH_2)_n-Ph$; wherein n is an integer from 2 to 4 and Ph represents the phenyl group.

9. A compound according to claim 8, wherein $R^1$ is hydrogen, methyl or hydroxymethyl.

10. The compound according to claim 9, wherein $R^1$ is methyl.

CLAIMS FOR AUSTRIA

1. A process for the preparation of an imidazole compound of the formula

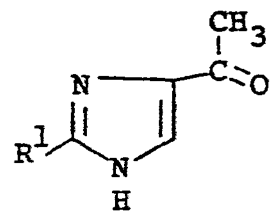

--- (I)

which comprises the steps of

(a) contacting an isoxazole compound of the formula

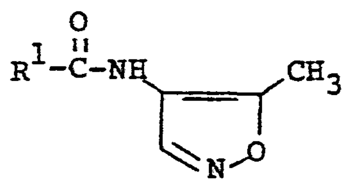

--- (II)

with hydrogen, in a reaction-inert solvent, in the presence of a catalytic amount of a hydrogenolysis catalyst, to give an intermediate compound of the formula

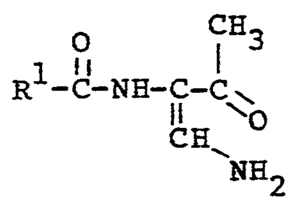

--- (III)

and (b) cyclizing the intermediate compound from step (a) by treating said intermediate compound with a basic agent having a $pK_b$ greater than 9, in a reaction-inert solvent;

wherein $R^1$ is hydrogen, alkyl having 1 to 6 carbons, hydroxymethyl or $-(CH_2)_n-Ph$, wherein n is an integer from 2 to 4 and Ph represents the phenyl group.

2. The process according to claim 1, wherein the basic agent used in step (b) is selected from the group consisting of alkali metal hydroxides, alkaline earth metal hydroxides, alkali metal alkoxides having 1 to 5 carbons, alkali metal hydrides and alkaline earth metal hydrides.

3. The process according to claim 2, wherein $R^1$ is hydrogen, methyl or hydroxymethyl.

4. The process according to claim 3, wherein step (a) is carried out under an atmosphere consisting substantially of hydrogen, at a temperature in the range from 0 to 80°C., at a pressure in the range from 0.5 to 10 $kg/cm^2$ and at a pH in the range from 4 to 8, and said hydrogenolysis catalyst is palladium, platinum or nickel.

5. The process according to claim 4, wherein the hydrogenolysis catalyst is present in an amount in the range from 1 to 10 weight-percent, based on the weight of said isoxazole.

6. The process according to claim 5, wherein the hydrogenolysis catalyst is palladium and it is suspended on powdered carbon, such that the ratio of catalyst to powdered carbon is in the range from 1:40 to 1:5 by weight.

7. The process according to claim 4, wherein step (b) is carried out at a temperature in the range from 60 to 120°C., and said basic agent used in step (b) is sodium hydroxide, potassium hydroxide or calcium hydroxide.

0156644

European Patent Office

# EUROPEAN SEARCH REPORT

Application number EP 85302066.7

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| D,A | US - A - 4 374 843 (LA MATTINA et al.) * Column 2, lines 44-68; column 6, lines 39-62 * | 1,8 | C 07 D 233/64<br>C 07 C 103/30 |
| P,A | US - A - 4 452 987 (LA MATTINA et al.) * Column 2, formula III, lines 58-68; column 3, lines 1-3; column 6, lines 15-64 * | 1,8 | |
| | | | **TECHNICAL FIELDS SEARCHED (Int. Cl 4)** |
| | | | C 07 D 233/00<br>C 07 C 103/00 |

The present search report has been drawn up for all claims

| Place of search | Date of completion of the search | Examiner |
|---|---|---|
| VIENNA | 04-07-1985 | BRUS |

**CATEGORY OF CITED DOCUMENTS**

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document